# EUROPÄISCHE PATENTSCHRIFT

(11) **EP 1 027 904 B1**
(45) Veröffentlichungstag und Bekanntmachung des Hinweises auf die Patenterteilung: **28.11.2001**
(21) Anmeldenummer: 99102518.0
(22) Anmeldetag: 10.02.1999
(51) Int. Cl.: A61M 16/04

(54) **Chirugisches Instrument für die Notfallmedizin**
First aid chirurgical instrument
Instrument chirurgical pour la médecine d'urgence

(43) Veröffentlichungstag der Anmeldung: 16.08.2000
(73) Patentinhaber: Klappenberger, J. Dr. med., 94327 Bogen (DE)
(72) Erfinder: Klappenberger, J. Dr. med., 94327 Bogen (DE)

(56) Entgegenhaltungen:
- DE-A- 3 335 715
- DE-A- 19 738 539
- DE-U- 29 723 780
- US-A- 3 476 113
- US-A- 4 291 690
- US-A- 5 681 323

## Beschreibung

Die Erfindung bezieht sich auf ein Koniotomiegerät für die Notfallmedizin, insbesondere eine sogen. Koniotomiegerät mit einem Trokar, einer in die Luftröhre einbringbaren Verweilkanüle und einer außen am Hals anliegenden Manschette.

Gattungsgemäße Koniotomiegeräte sind an sich bekannt. So offenbart z.B. die DE 88 85 715 C7 ein solches Gerät, bei dem eine am Hals zu positionierende Manschette mit einem unter 45° gegenüber der Mmanschettenoberfläche geneigten Flansch mit einem Befestigungsteil ausgerüstet ist, in das ein axial geführtes Kathetersystem mit Kanüle und Mandrin einsetzbar ist und, nach erfolgtem Einführen in den Atmungstrakt mittels einer Klemmsicherung am Befestigungsteil zu arretieren ist.

Weiter ist der US PS 8 906 956 ein solches Instrument zu entnehmen, das eine L-förmige Konfiguration aufweist und eine Öffnung für einen Trokar besitzt.

Auch die US PS 4 291 690 offenbart ein ähnliches Instrument welches eine selbstlimitierende Eindringtiefe bis in das hintere Drittel der Trachea aufweist und, beidseitig von einem ovalen Flansch ausgehend, rohrförmige Stutzen für die Hindurchführung eines in die Traches einzuführenden Luftschlauches besitzt.

Schließlich sind ähnliche Instrumente noch aus den beiden US PS 34 76 112 und 29 23 299 und der DE PS 19 514 433 bekannt geworden.

Es liegt auf der Hand, das diese bekannten Koniotomiegeräte mit erheblichen Nachteilen behaftet sind. Für den Einsatz bei Notfällen sind sie zu kompliziert aufgebaut, bestehen aus zu vielen vor dem Eingriff zu montierenden Einzelteilen und sind außerdem in der Handhabung selbst für einen erfahrenen Arzt in derartigen Situationen zu umständlich, wodurch viel, für die Lebensrettung evtl. entscherdende, Zeit verloren gehen kann. Die mit aufwendiger Mechanik und mit gebogenen in die Trachea einzuführenden Kanülen ausgerüsteten Instrumente lassen sich in der Praxis kaum einsetzen, insbesonder diejenigen mit gebogenen Kanülen sind für die Notfallmedizin völlig ungeeignet.

Hier setzt die Erfindung ein. Der Erfindung, wie sie in den Anspruchen beschrieben ist, liegt die Aufgabe zugrunde, ein Koniotomiegerät zu schaffen, das einfach im Aufbau, schnell und leicht zu handhaben ist. Sichtkontakt in die Trachea möglich ist und, bei Bedarf, d.h. bei schweren Verletzungen beispielsweise im Kieferbereich, das Entweichen von in die Trachea eingebrachter Luft durch den oberen Teil des Kehlkopfes mit Sicherheit verhindert und.

Die mit der Erfindung erzielten Vorteile sind insbesondere der einfache Aufbau des nur aus zwei Telen bestehenden Koniotomiegerätes mit innerer und außerer Selbstfixterung durch den endständigen, umlaufenden Wulst der Verweilkanule, bzw. den seitlichen Fixierflügeln des Basisteiles und, wegen der geraden Form, dessen schnelle und einfache Handhabung, was insbesondere in außersten Notfällen wichtig ist. Ferner keinerlei Kontakte des Instruments mit der Innenwand der Traches, was reflektorische Verkrampfungen weitgehend ausschließt. Ein weiterer Vorteil ist darin zu sehen, daß die Messerform, d.h. Schneide mit senkrechtem Verlauf, ein Durchtrennen der elastischen Fasern des lig. conicum verhindert, was später notwendig werdende plastische Operationen überflüssig macht. Von großem Vorteil ist außerdem die Möglichkeit der Sichtkontrolle der Trachea durch die kurze und gerade Form der Verweilkanule und, daß durch die Möglichkeit einen kleinen Verschlußbeutel in die Trachea mit einzubringen, das Entweichen von eingeblasener Luft nach oben verhinderbar ist

Schließlich gestaltet sich das Einbringen der Blockierkanüle in das Basisteil völlig problemlos.

Die Erfindung ist nachstehend anhand eines in den Abbildungen dargestellten Ausführungsbeispieles näher erläutert.

Es zeigt :
Fig. 1 einen Horizontalschnitt durch das Koniotomiegerät mit Trokar und

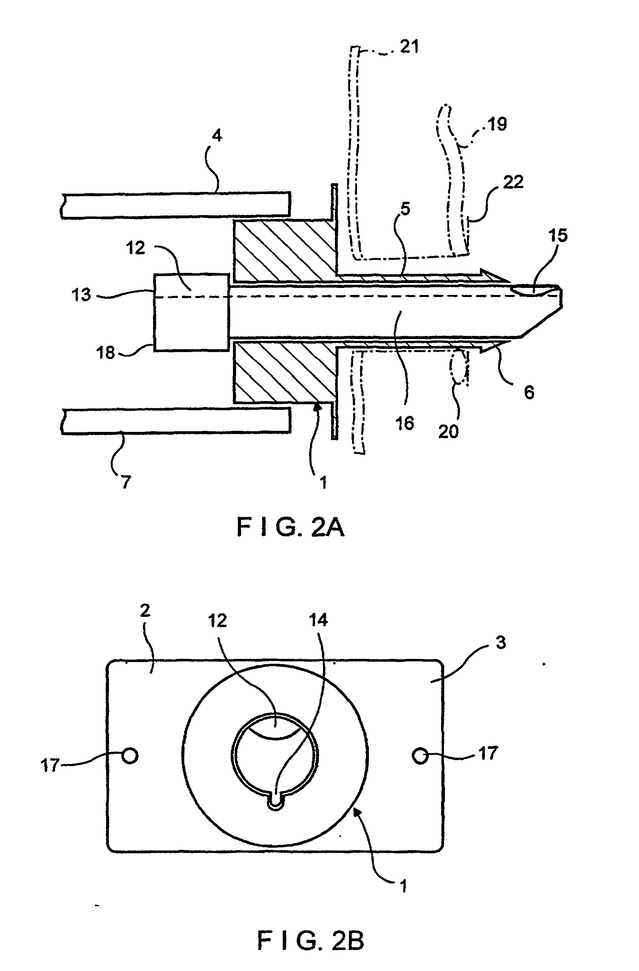

Fig. 2 einen Vertikalschnitt der Fig. 1 mit Blockierkanüle, jeweils mit Draufsicht.

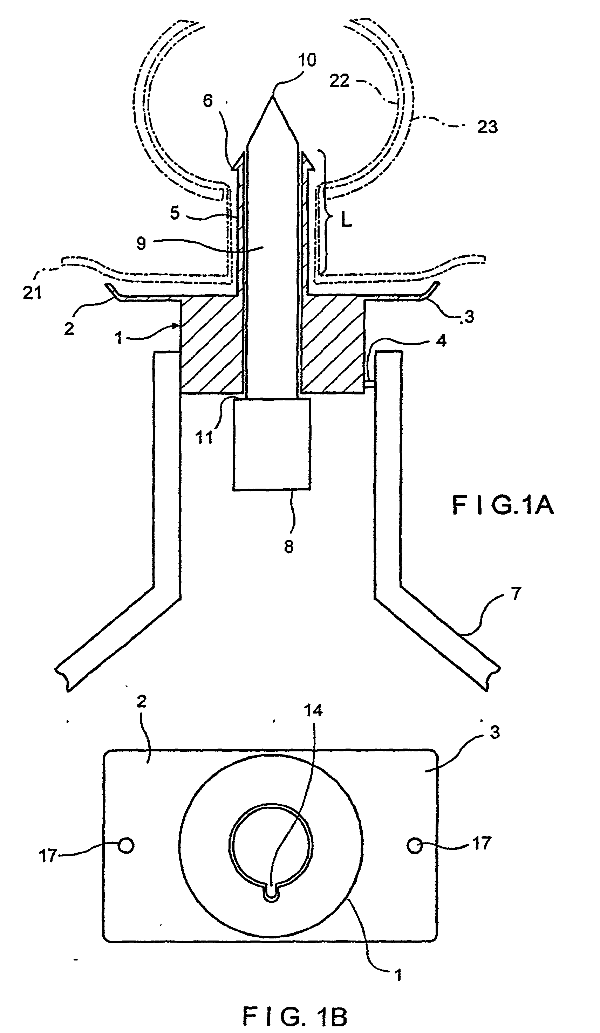

Wie aus der Figur 1 ersichtlich, besteht das Koniotomiegerät im wesentlichen aus nur zwei Teilen, nämlich dem Basisteil 1 und dem Trokar 8. Das Basisteil 1 ist mit zwei Fixierflügeln 2 und 3 versehen mit denen es außen auf der Halspartie des Patienten fixiert wird. Mit 17 ist je eine Öse bezeichnet. Das Basisteil 1 weist außerdem einen Ansatz 4 für den nur andeutungsweise dargestellten sogen. Ambubeutel, einen Luftbalg 7 auf und eine in die Trachea 23 des Patienten einzuführende Verweilkanüle 5. Die Verweilkanüle 5 ist kurz ausgebildet und besitzt einen endständigen umlaufenden Fixierwulst 6, der ein Herausrutschen des Koniotomiegerätes nach dessen Einführen in die Trachea 23 verhindert Somit ist das Gerät sowohl von außen, als auch von innen fixiert. Der Trokar 8 besitzt eine skalpellartige Schneide 10 mit senkrechtem Verlauf und ggfl. einen Anschlag 11 mit dem die Eindringtiefe des Trokars 8 begrenzt werden kann.

Zum besseren Verständnis ist in den Abbildungen der Schildknorpel mit 19, der Ringknorpel mit 20, die Haut mit 21 und Schleimhaut der Trachea 23 mit 22 bezeichnet.

Es hat sich als vorteilhaft erwiesen, die optimals Länge L der Verweilkanüle 5 zwischen dem Basisteil 1 und deren Ende auf etwa 17 mm zu begrenzen und zwar incl. dem endständigen Wulst, der ca. 1 mm Dicke aufweisen soll. Für spezielle Anwendungen kann die Verweilkanüle 5 beispielsweise auch teleskopisch ausgebildet sein, um sie bei Bedarf in ihrer Länge verstellen zu können. Der Innendurchmesser der Verweilkanüle 5 ist mit 4-6 mm ausreichend bemessen, wobei 6 mm Durchmesser für große Erwachsene vorgesehen ist. Um ein Verdrehen des Trokars 8 zu verhindern ist weiter eine Fixiernut und -feder 14 mindestens in einem Teilabschnitt der Verweilkanüle 5 und dem Trokar 8 bzw. dessen Schaft 9 vorgesehen. Hierdurch wird beim richtigen Aufsetzen des Basisteils 1 der senkrechte Verlauf der skalpellartigen Schneide (10) des Trokars (8) gesichert.

Eine Weiterbildung der Erfindung sieht vor, in den Durchtrittskanal der Verweilkanüle 5 eine Blockierkanüle 18 einzuführen, die zwei getrennte Luftkanäle besitzt. Der eine Luftkanal 12 ist außen mit einer Verschlußmembran 13 versehen und mündet innen in einen in dessen Ende angeordneten Verschlußbeutel 15. Dieser Verschlußbeutel 15 bläht sich bei Bedarf dann durch Druck auf die Verschlußmembran 13 in die Trachea oberhalb der Luftzufuhrleitung 16 auf und verhindert so ein Entweichen der durch diese Luftzufuhrleitung 16 in die Trachea 23 eingeblasenen Luft nach cranial.

Die Verweilkanüle 5, die gerade und sehr kurz ausgebildet ist, gibt dem behandelnden Arzt zusätzlich die Möglichkeit eines ausreichenden Sichtkontaktes in die Trachea T, wobei, nach Entfernen des Trokars 8, auch ein in den Abbildungen nicht dargestelltes, dünnes Glasfaserkabel zusätzlich eine Beleuchtung der Innenwand der Trachea ermöglicht. Beispielsweise kann auch durch die Verweilkanüle 5 auch ein Absauggerät oder dergl. eingeführt werdenn. Auch photographische Aufnahmen sind mittels eines durch die Verweilkanüle 5 gesteckten Endoskops etc. möglich. Schließlich kann der Fixierwulst 6 als Beleuchtungskörper für die Trachea T ausgebildet sein. Der Einsatz solcher Absaug-. Beleuchtungs- und/oder Sichtmittel etc.wird der behandelnde Arzt zwar nur von Fall zu Fall einsetzen müßen, die erforderlichen Maßnahmen zur Durchfuhrung entsprechender Verrichtungen sind jedoch bereits bei der Herstellung des Koniotomiegerätes in dieses integrierbar.

Die Anwendung des Koniotomiegerätes gestaltet sich äußerst einfach, sodaß das Gerät sowohl von Ärzten als auch von Sanitätern und sonstigen Hilfspersonal völlig unproblematisch eingesetzt werden kann. Bei Intubationshindernissen die eine orotracheale Intubation unmögllich machen, kann mit dem Koniotomiegerät gem. der Erfindung ein Zugang in die Trachea des Patienten gefunden werden. Dabei wird wie folgt vorgegangen:
Mit dem linken Zeigefinger tastet sich der behandelnde Arzt am Schildknorpel bis zu dessen unteren Rand entlang, sodaß die Fingerkuppe am lig. conicum aufliegt in diese dabei entstehende Mulde wird die Spitze des Koniotomiegerätes auf die Haut gesetzt und senkrecht zur Körperoberfläche in den Trachealraum gestoßen. Dabei wird vorher der Zeigefinger zurückgezogen. Der Daumen und Mittelfinger fixiert zugleich den Kehlkopf des Patienten. Nach dem Einstoßen der skalpellartigen Schneide 10 wird beim Durchdringen der elastischen Membran ein leichter Widerstand spürbar, wenn der Fixierwulst 6 diese Membran durchdringt. In diesem Augenblick hat die Spitze des Trokars noch einen Abstand von etwa 7 mm zur Hinterwand der Trachea. Dadurch besteht nur ein minimales Risiko die hintere Wand der Trachea zu verletzen. Nach Entfernen des Trokars 8 kann dann sofort mit einem Luftbalg 7, bzw. einem sogen. "Ambubeutel" etc. mit der Beatmung des Patienten begonnen werden.

Die Erfindung ist selbstverständllich nicht auf die Anwendung bei verschlossenem Atmungstrakt im Rachenbereich und den HNO-Bereich beschränkt. Das Instrument kann außerdem bei diversen anderen Anwendugen in der Chirurgie mit Erfolg eingesetzt werden.

### Stückliste

- 1: Basisteil
- 2: Fixierflügel 1
- 3: Fixierflügel r
- 4: Anschlußstück für Ambubeutel
- 5: Verweilkanüle
- 6: Fixierwulst an Verweilkanüle
- 7: Ambubeutel
- 8: Trokar
- 9: Schaft des Trokars
- 10: Schneide des Trokars
- 11: Anschlag am Trokar bzw. Blockierkanüle
- 12: Luftkanal für Verschlußbeutel
- 13: Verschlußmembran
- 14: Fixieraut und Fixierfeder
- 15: Verschlußbeutel
- 16: Luftzufuhrleitung
- 17: Halteose
- 18: Blockierkanüle
- 19: Schildknorpel
- 20: Ringknorpel
- 21: Haut
- 22: Schleimhaut
- 23: Trachea

## Patentansprüche

1. Koniotomiegerät für die Notfallbeatmung mit einem durch eine in die Trachea (23) einführbare Verweilkanüle (5) geführten Trokar (8) und mit einer außen am Hals anzulegenden Manschette für die Befestigung des vome am Hals des Patienten anzusetzenden Basisteils (1) des Gerätes, wobei das Basisteil (1) an seinem vorderen, halsnahen Ende seitliche Fixierflügel (2,3) und am trachealen Ende eine Fixiereinrichtung aufweist, die Verweilkanüle (5) auf ihrer gesamten Länge als Durchtrittskanal für den Schaft (9) des Trokars (8) ausgebildet ist, wobei dessen Schaft (9) mit einer skalpellartiger Schneide (10) versehen ist und das Basisteil (1) mit seiner Verweilkanüle (5) senkrecht auf den Hals des Patienten aufsetzbar und die Verweilkanüle (5) in dessen Trachea (23) ebenfalls senkrecht einführbar ist, und an ihrem rückwärtigen, halsfemen Ende ein Anschlußstück (4) für einen Luftbalg (7) besitzt, **dadurch gekennzeichnet, daß** die zentral vom Basisteil (1) nach vorne in Richtung des Halses des Patienten hervor ragende gerade Verweilkanüle (5) einen endständigen fest angeformten umlaufenden Fixierwulst (6) aufweist und die skalpellartige Schneide (10) des Trokars (8) von cranial nach caudal und damit parallel zu den elastischen Fasern des ligamentum conicum verläuft.

2. Koniotomiegerät für die Notfallmedizin nach Anspruch 1, **dadurch gekennzeichnet, daß,** die Länge (L) des in den Körper des Patienten eingeführten Abschnittes der Verweilkanüle (5) etwa 17 mm, der Innendurchmesser der Verweilkanüle (5) etwa 4 - 6 mm und deren Fixierwulst (6) eine radiale Dicke von etwa 1 mm aufweist.

3. Koniotomiegerät für die Notfallmedizin nach Anspruch 1 oder 2, **dadurch gekennzeichnet, daß** eine Blockierkanüle (18) durch die Verweilkanüle (5) in die Trachea (23) einbringbar ist durch deren Luftkanal (12) ein Verschlußbeutel (15) für die Trachea (23) auftreibbar ist.

4. Koniotomiegerät für die Notfallmedizin nach Anspruch 3, **dadurch gekennzeichnet, daß** die Blockierkanüle (18) einen Luftkanal (12) und eine von diesem getrennte Lukftzufuhrleitung (16) aufweist, der Luftkanal (12) am halsfemen Ende mittels einer Verschlußembran (13) verschlossen ist, und am patientenseitigen Ende in einen Verschlußbeutel (15) mündet.

5. Koniotomiegerät für die Notfallmedizin nach einem der Ansprüche 1 bis 4, **dadurch gekennzeichnet, daß** die Schneide (10) des Trokars (8) senkrecht zum Ringknorpel (20) vorgesehen ist und nicht mehr als 7 mm über den Fixierwulst (6) hervorragt.

6. Koniotomiegerät für die Notfallmedizin nach einem der Ansprüche 1 bis 5, **dadurch gekennzeichnet, daß** der Schaft (9) des Trokars (8) einen Anschlag (11) aufweist.

7. Koniotomiegerät für die Notfallmedizin nach Anspruch 6, **dadurch gekennzeichnet, daß** der Anschlag (11) auf dem Schaft in dessen axialer Richtung justierbar ist.

8. Koniotomiegerät für die Notfallmedizin nach einem der Ansprüche 1 bis 7, **dadurch gekennzeichnet, daß** die Verweilkanüle (5), der Trokar (8) und die Blockierkanüle (18) eine Fixiemut und -feder (14) aufweisen.

9. Koniotomiegerät für die Notfallmedizin nach Anspruch 1, **dadurch gekennzeichnet, daß** Absaug-, Sicht- und Beleuchtungsmittel für die Trachea (23) in die Verweilkanüle einführbar sind.

## Claims

1. Coniotomy device for emergency respiration with a trocar (8) inserted through an indwelling cannula (5) that can be inserted into the trachea (23) and with a cuff to be positioned externally on the neck for attaching the base part (1) of the device to be positioned at the front of the neck of the patient, with the base part (1) having lateral fixation wings (2, 3) at its front end near the neck and a fixation device at the tracheal end, the indwelling cannula (5) being designed over its entire length as a passage channel for the shaft (9) of the trocar (8), with this shaft (9) having a scalpel-like cutting edge (10) and the base part (1) with its indwelling cannula (5) being able to be positioned vertically on the neck of the patient and the indwelling cannula (5) also being able to be inserted vertically in the patient's trachea (23), and having a fitting (4) for air bellows (7) at the rear end remote from the patient's neck, **characterised in that** the straight indwelling cannula (5) projecting centrally from the base part (1) forwards in the direction of the neck of the patient has a circular fixation bead (6) permanently moulded onto its end and the scalpel-like cutting edge (10) of the trocar (8) runs from cranial to caudal and thus parallel to the elastic fibres of the ligamentum conicum.

2. Coniotomy device for emergency medicine in accordance with Claim 1, **characterised in that** the length (L) of the section of the indwelling cannula (5) inserted into the body of the patient is about 17 mm, the internal diameter of the indwelling cannula (5) is about 4 - 6 mm and its fixation bead (6) has a radial thickness of about 1 mm.

3. Coniotomy device for emergency medicine in accordance with Claim 1 or 2, **characterised in that** a blocking cannula (18) can be inserted through the indwelling cannula (5) into the trachea (23) and that through its air channel (12) a closure bag (15) for the trachea (23) can be inflated.

4. Coniotomy device for emergency medicine in accordance with Claim 3, **characterised in that** the blocking cannula (18) has an air channel (12) and an air supply line (16) separate from said air channel, the air channel (12) being closed at the end remote from the patient's neck by means of a closure membrane (13), and leading into a closure bag (15) at the patient end.

5. Coniotomy device for emergency medicine in accordance with one of Claims 1 to 4, **characterised in that** the cutting edge (10) of the trocar (8) is intended to be positioned vertically with respect to the cricoid cartilage (20) and does not project beyond the fixation bead (6) by more than 7 mm.

6. Coniotomy device for emergency medicine in accordance with one of Claims 1 to 5, **characterised in that** the shaft (9) of the trocar (8) has a limit stop (11).

7. Coniotomy device for emergency medicine in accordance with Claim 6, **characterised in that** the limit stop (11) on the shaft is adjustable in its axial direction.

8. Coniotomy device for emergency medicine in accordance with one of Claims 1 to 7, **characterised in that** the indwelling cannula (5), the trocar (8) and the blocking cannula (18) have a fixation tongue and groove (14).

9. Coniotomy device for emergency medicine in accordance with Claim 1, **characterised in that** suction, inspection and illumination equipment for the trachea (23) can be inserted into the indwelling cannula.

## Revendications

1. Instrument de trachéotomie pour la respiration d'urgence avec un trocart (8) inséré dans une canule à demeure (5) qui peut être introduite dans la trachée (23) et avec une manchette à appliquer à l'extérieur sur la gorge pour la fixation de l'élément de base (1) de l'instrument à placer à l'avant sur la gorge du patient, l'élément de base (1) présentant des ailettes de fixation (2, 3) sur son extrémité avant proche de la gorge et un dispositif de fixation sur son extrémité trachéale et la canule à demeure (5) étant conçue comme canal de passage sur la totalité de sa longueur pour la tige (9) du trocart (8), la tige (9) de ce dernier étant munie d'une lame de type scalpel (10) et l'élément de base (1) avec sa canule à demeure (5) pouvant être placé verticalement sur la gorge du patient, la canule à demeure (5) pouvant également être introduite verticalement dans la trachée (23) de ce dernier et possédant, sur sa partie arrière éloignée de la gorge, une pièce de jonction (4) pour un ballon à air (7), **caractérisé en ce que** la canule à demeure (5) droite qui émerge du centre de l'élément de base (1) vers l'avant en direction de la gorge du patient présente à son extrémité un bourrelet de fixation (6) circulaire intégré et que la lame de type scalpel (10) du trocart (8) est placée à l'horizontale, c'est à dire en parallèle aux fibres élastiques du ligamentum conicum.

2. Instrument de respiration d'urgence pour la médecine d'urgence selon la revendication 1, **caractérisé en ce que** la longueur (L) de la partie de la canule à demeure (5) introduite dans le corps du patient est d'environ 17 mm, le diamètre intérieur de la canule à demeure (5) est d'environ 4 - 6 mm et que son bourrelet de fixation (6) présente une épaisseur radiale d'environ 1 mm.

3. Instrument de respiration d'urgence pour la médecine d'urgence selon la revendication 1 ou 2, **caractérisé en ce qu'**une canule de blocage (18) peut être introduite dans la trachée (23) à travers la canule à demeure (5), le canal d'air (12) de la canule de blocage permettant d'introduire un ballon de fermeture (15) pour la trachée (23).

4. Instrument de respiration d'urgence pour la médecine d'urgence selon la revendication 3, **caractérisé en ce que** la canule de blocage (18) présente un canal d'air (12) et une conduite d'amenée d'air (16) séparée de ce dernier, que le canal d'air (12) est fermé à l'aide d'une membrane de fermeture (13) à son extrémité opposée à la gorge et débouche dans un ballon de fermeture (15) par son extrémité située du côté du patient.

5. Instrument de respiration d'urgence pour la médecine d'urgence selon les revendications 1 à 4, **caractérisé en ce que** la lame (10) du trocart (8) est placée à la verticale par rapport au cartilage cricoïde (20) et ne dépasse pas de plus de 7 mm au-dessus du bourrelet de fixation (6).

6. Instrument de respiration d'urgence pour la médecine d'urgence selon les revendications 1 à 5, **caractérisé en ce que** la tige (9) du trocart (8) présente une butée (11).

7. Instrument de respiration d'urgence pour la médecine d'urgence selon la revendication 6, **caractérisé en ce que** la butée (11) peut être ajustée sur la tige dans sa direction axiale.

8. Instrument de respiration d'urgence pour la médecine d'urgence selon les revendications 1 à 7, **caractérisé en ce que** la canule à demeure (5), le trocart (8) et la canule de blocage (18) présentent une rainure et un ressort de fixation (14).

9. Instrument de respiration d'urgence pour la médecine d'urgence selon la revendication 1, **caractérisé en ce que** les outils d'aspiration, les outils optiques et d'éclairage pour la trachée (23) peuvent être introduits dans la canule à demeure.
